# EUROPEAN PATENT APPLICATION

(11) **EP 1 849 403 A1**
(43) Date of publication of application: **31.10.2007**
(21) Application number: 07008580.8
(22) Date of filing: 26.04.2007
(51) Int. Cl.: A61B 1/12, A61B 19/00, A61L 2/16, A61L 2/18, A46B 5/06, B08B 9/04, A61L 2/24

(54) **Endoscope washer-disinfector and brush cassette detachably loaded to the same**

(30) Priority: 26.04.2006 JP 2006122392
(71) Applicant: OLYMPUS MEDICAL SYSTEMS CORP., Shibuya-ku Tokyo 151-0072 (JP)
(72) Inventor: Kobayashi, Kenichi, Shibuya-ku Tokyo 151-0072 (JP); Suzuki, Eiri, Shibuya-ku Tokyo 151-0072 (JP); Suzuki, Shintaro, Shibuya-ku Tokyo 151-0072 (JP); Hasegawa, Hitoshi, Shibuya-ku Tokyo 151-0072 (JP); Ogawa, Akihisa, Shibuya-ku Tokyo 151-0072 (JP); Noguchi, Toshiaki, Shibuya-ku Tokyo 151-0072 (JP)
(74) Representative: von Hellfeld, Axel

(57) **Abstract**

An endoscope washer-disinfector cleans and disinfects an endoscope with a duct. A brush cassette is detachably loaded to a loading part of the washer-disinfector and comprises a brush element and a shaft holding the brush element. Inside the cassette, first and second members are formed to hold the shaft tight therebetween for making the shaft advance and retreat to and from the duct. A lock device separates the second member from the first member so that the second member is located at a first position. The lock device locks the separated second member, when the brush is not loaded on the loading part. When the cassette is loaded to the loading part, the lock device also unlocks the locked second member responsively to an operating force and allows the second member to be moved to a second position where the first and second members hold tight the shaft.

## Description

### CROSS REFERENCE TO RELATED APPLICATION

The patent application related to and incorporates by reference Japanese Patent application No. 2006-122392 filed on April 26, 2006.

### Background of the Invention

### (The Field of the Invention)

The present invention relates to an endoscope washer-disinfector capable of cleaning (or washing) ducts of an endoscope and a brush cassette detachably loaded to the endoscope washer-disinfector.

### (Related Art)

In medical therapy, one helpful means for inspecting and curing body cavities is to use an endoscope. The endoscope includes an insertion tube to be inserted to the body cavities. This insertion tube has various kinds of ducts (i.e., endoscopic channels or simply channels), such as a therapeutic-instrument insertion duct which also serves as a suction duct, a gas-feed and water-feed duct, and a forward water-feed duct, which are formed to run through the tube. Thus, when an endoscope is used once, body fluid and feculence adhere on the external surface of the insertion tube but also inside areas of the ducts of the insertion tube. Therefore, after use, the external surface and ducts of the insertion tube need to be thoroughly cleaned and disinfected. To this end, in recent years, an endoscope cleaning and disinfecting apparatus has been put into practical use, which can automatically clean and disinfect the endoscope even in its respective ducts.

Further, pieces of tissue or other substances removed from body cavities in inspecting or making treatment with the use of the endoscope pass through the ducts of the endoscope. Especially, a therapeutic-instrument insertion duct also serving as a sucking duct is subjected to such passing. Thus, feculence easily adheres onto the ducts and is hard to be removed merely in a cleaning process conducted with the endoscope cleaning and disinfecting apparatus.

To increase cleaning capabilities for the various ducts of the endoscope, in general practice, an operator performs preliminary cleaning prior to conducting the processing (cleaning and disinfecting) steps with the use of an endoscope cleaning and disinfecting apparatus. Practically, the operator inserts a cleaning brush, including a brush chip fixed to a distal end of, for instance, an elongated shaft, into various ducts of an endoscope for rubbing and cleaning the same. This allows the ducts to be preliminarily cleaned, thereby removing feculence from the ducts.

However, the above manual preliminary cleaning (i.e., manually inserting the cleaning brush into respective endoscope ducts and manually rubbing and cleaning the ducts) causes the operator to bear troublesome work. This preliminary cleaning process results in an increase in work time required for the endoscope to be clean and disinfected.

In view of these situations, there has been known an endoscope cleaning and disinfecting apparatus for automatically cleaning ducts of the insertion tube of an endoscope. In such an apparatus, a cleaning brush is automatically inserted into each duct being cleaned, and then made to go backward and forward along inside the duct. This way of automatic cleaning is a relatively easier and quicker manner.

This kind of endoscope cleaning and disinfecting apparatus is provided with a brush cassette dedicated to such automatic cleaning. This brush cassette comprises a rotating roller not only for feeding the cleaning brush into each duct of an endoscope but also pulling back the cleaning brush from the duct. The rotating roller is driven by an electric motor to be driven electrically.

In particular, as an apparatus for ducts of endoscopes with the use of brushes, a cleaning apparatus provided by Japanese Patent Laid-open Publication No. 8-252219 is known. This cleaning apparatus is equipped with a rotating roller and an auxiliary roller (serving as a guide member) which allow a cleaning brush to be inserted and pulled back into and from each duct. Between both the rotating and auxiliary rollers, the shaft of the cleaning brush is held tight for being guided. Both the rollers are made of, for example, rubber, because the shaft needs to have good friction performance. When the rotating roller is driven, the shaft, which is guided between both rollers, can be moved backward and forward. Thus the brush, mounted to the distal end of the shaft, can be made to advance and retreat along within each endoscope duct, thereby allowing the duct to be brush-cleaned.

In the cleaning apparatus described above, in cases where the cleaning brush is not in work (that is, the cleaning brush is not subjected to its advancing and retreating work), electromagnetic valves lock the rotating and auxiliary rollers so that the rollers cannot rotate. This locking mechanism prevents the cleaning brush from moving improperly.

However, the structure in which the shaft of the cleaning brush is held tight between the rotating roller and the guide member (auxiliary roller) suffers another problem. This problem results from the fact that the rotating roller and the guide member always hold the shaft tightly such that both rollers are constantly pressed from each other. Due to this constant pressing, the rotating roller and/or the guide member may be deformed during transportation or storage of the brush cassette (that is, in such conditions, the cassette has not been used for a long time). This deformation, if happened, brings about a concern that the cleaning brush cannot be moved smoothly backward and forward in cleaning.

Another problem of the cleaning apparatus listed in the publication comes from the structure in which the rotating roller and the guide member are arranged within the brush cassette. Hence it is impossible to visually observe the forgoing deformations which may occur inside the cassette. Another problem is that it is difficult to visually determine after use whether the cleaning brush is a new one or a used one, because pulling back the brush from the cassette after a cleaning work creates the same appearance as that of the cassette which has not been in use yet, i.e., which is new.

### Summary of the Invention

The present invention has been made in consideration of the foregoing conventional situations, and has an object to .provide an endoscope washer-disinfector and a brush cassette detachably loaded to this endoscope washer-disinfector, which are able to secure a good cleaning performance by removing the affects depending on a period of time during which the brush cassette has not been in use, even if such a non-use period lasts long.

In order to achieve the above object, an endoscope washer-disinfector according to the present invention is provided to, at least, clean (or wash) and disinfect an endoscope with a duct. The apparatus comprises a frame having a bath used for cleaning and disinfection of the endoscope accommodated in the bath and a brush cassette detachably loaded to a loading part of the frame. The brush cassette is formed to comprise a brush element cleaning the duct of the endoscope, a shaft connected with the brush element; a first member and a second member formed to hold the shaft tight therebetween and to make the tight held shaft advance and retreat to and from the duct, and a lock device. This lock device has the capabilities of i) separating the second member from the first member so that the second member is located at a first position and ii) locking the separated second member, when the brush cassette is not loaded on the loading part, while iii) unlocking the locked second member in response to an operating force given responsively to a loading action given to the brush cassette and iv) allowing the second member to be moved to a second position where the first and second members hold tight the shaft therebetween, when the brush cassette is loaded to the loading part. The endoscope washer-disinfector also comprises an unlocking commander unlocking the locked second member by providing the operating force to the lock device when the brush cassette is loaded to the loading part.

In addition, to achieve the foregoing object, as another aspect of the present invention, there is provided a brush cassette detachably loaded to a loading part of an endoscope washer-disinfector for, at least, cleaning and disinfecting an endoscope with a duct. The brush cassette comprises brush element cleaning the duct of the endoscope, a shaft connected with the brush element; a first member and a second member formed to hold the shaft tight therebetween and to make the tight held shaft advance and retreat to and from the duct, and a lock device. This lock device has the capabilities of i) separating the second member from the first member so that the second member is located at a first position and ii) locking the separated second member, when the brush cassette is not loaded on the loading part, while iii) unlocking the locked second member in response to an operating force given responsively to a loading action given to the brush cassette and iv) allowing the second member to be moved to a second position where the first and second members hold tight the shaft therebetween, when the brush cassette is loaded to the loading part.

Accordingly, in the endoscope washer-disinfector and the brush cassette according to the present invention, the second member Is located at the first position separated from the first position and locked thereat, when the brush cassette is not loaded on the loading part of the apparatus. Thus the shaft is not subjected to any pressing between the first and second members at all. The shaft intervening between the first and second members is therefore avoidable from being deformed. Thus, in using the brush cassette, the cleaning performance has no affections which result from a long-term non-use of the brush cassette. Thus a cleaning performance can still be maintained high.

Meanwhile, when the brush cassette is loaded to the loading part, the foregoing locked state is released in response to an operating force arising in association with the loading action. This unlocking action will cause the second member to move to the second position, whereby the first and second members hold the shaft tightly therebetween so as to make the shaft advance and retreat therealong.

### Brief Description of the drawings

In the accompanying drawings:
Fig. 1 is a perspective view showing an endoscope washer-disinfector r according to a first embodiment of the present invention, together with an endoscope mounted in a cleaning and disinfecting bath of the washer-disinfector, in which a top cover thereof is opened;
Fig. 2 is a perspective view showing the endoscope washer-disinfector, showing another state in which the top cover thereof is closed;
Fig. 3 is a perspective view showing the entire appearance of a brush cassette to be mounted to a loading part of a frame composing part of a main body unit of the endoscope washer-disinfector;
Fig. 4 is a perspective view showing an inner structure of the brush cassette as well as the motions of a guide member arranged in the cassette;
Fig. 5 is a partial perspective view of the loading part arranged to the frame of the main body unit;
Fig. 6 is a perspective view showing the inner structure of the brush cassette as well as the motions of the guide member arranged in the cassette;
Fig. 7 is a partial disassembled perspective view outlining a state in which the brush cassette is on the way to loading to the loading part;
Fig. 8 is a partial perspective view outlining the inner structure as well as the motions of the guide member arranged within the brush cassette;
Fig. 9A illustrates a problem associated with a conventional structure of a brush cassette;
Fig. 9B illustrates an advantage obtained by the embodiment according to the present invention;
Fig. 10 is a perspective view showing an inner structure of a brush cassette as well as the motions of a guide roller within the cassette, according to a second embodiment of the present invention;
Fig. 11 is a partial perspective view showing a loading part arranged with a main body unit of an endoscope washer-disinfector according to the second embodiment;
Fig. 12 is a perspective view showing the inner structure of the brush cassette as well as the motions of the guide roller;
Figs. 13 and 14 are partial perspective views explaining the motions of the rollers within the brush cassette in association with a loading part according to the second embodiment;
Figs. 15 - 17 are partial sectional views illustrating the motions of the guide roller within the brush cassette in association
Fig. 18 is a perspective view showing an inner structure of a brush cassette loaded to an endoscope washer-disinfector, together with a control system for the apparatus, according to a third embodiment of the present invention; and
Fig. 19 is a sectional view explaining the motions of a guide roller according to the third embodiment.

### Detailed Description of the Preferred Embodiments

With reference to the accompanying drawings, various embodiments of an "endoscope washer-disinfector" (or called an "endoscope cleaning and disinfecting apparatus") according to the present invention will now be described.

### (First embodiment)

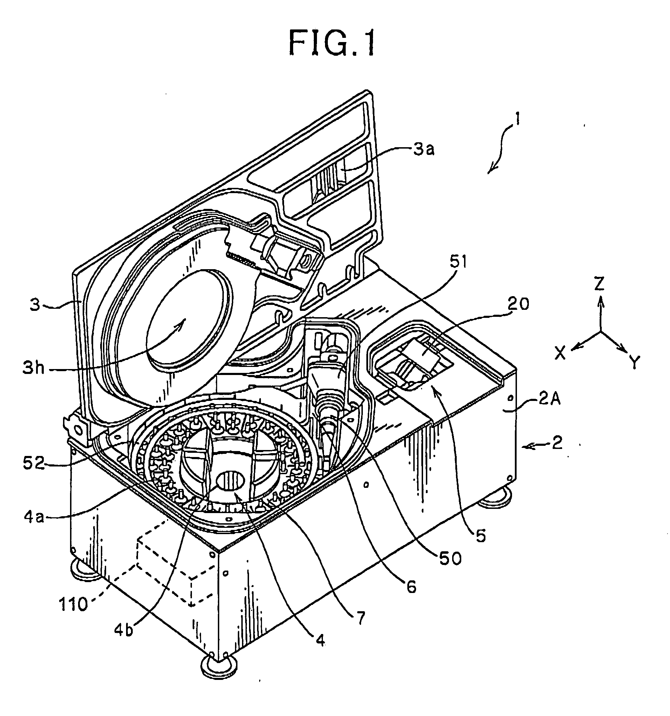

First, referring to Figs. 1-9, a first embodiment of the endoscope washer-disinfector according to the present invention will now be described.

Fig. 1 shows an outlined appearance of the endoscope washer-disinfector 1 according to the present embodiment. As shown, the endoscope washer-disinfector 1 is constructed to have a main body unit 2, a top cover 3 serving as a cover member which can be opened and closed freely from and to the upper face of the main body unit 2, and a brush cassette 20 detachable to and from the main body unit 2 and formed for cleaning an endoscope 50. The main body unit 2 comprises, as its casing, a frame 2A which is formed into a substantial box shape. Inside the frame 2A, there are provided various mechanical and electric mechanisms for cleaning and disinfecting endoscopes. In the present embodiment, the terms "cleaning (or washing)" and "disinfecting" represent in meaning a term "processing" which includes various other steps, such as rinsing and drying.

At the upper face part of the frame 2A, there are formed a cleaning and disinfecting bath (hereinafter, simply referred to as "cleaning bath") in which the endoscope 50 is accommodated for cleaning and disinfection and a loading part 5 to which the forgoing brush cassette 20 is detachably loaded (hereinafter the loading part is referred to as "loading part."

The cleaning bath 4 includes a manipulating-member accommodating bath 6 and an insertion-tube accommodating bath 7, which compose a main part of the bath. The manipulating-member accommodating bath 6 is shaped in accordance with the shape of a manipulating unit 51 of the endoscope 50. The insertion-tube accommodating bath 7 is shaped to accommodate therein an insertion tube 52 of the endoscope 50, with the insertion tube 52 wound in the bath 7.

The insertion-tube accommodating bath 7 is provided with a plurality of support members 4a and a cleaning-case accommodating portion 4b. The support members 4a are used to support the wound accommodated insertion tubes 52 so that the tubes 52 are mutually separated and located at predetermined intervals. The cleaning-case accommodating portion 4b, which is located at the center of the bath 4, supports a not-shown cleaning case. This cleaning case accommodates therein various accessories, such as buttons and forceps taps, of the endoscope 50 and is used to clean and disinfect such accessories together with the endoscope 50 itself.

The loading part 5 is located near the manipulating-member accommodating bath 6 of the cleaning bath 4. The brush cassette 20 is detachably mounted at the loading part 5 such that the cassette is for cleaning ducts formed in the insertion tube 52 along its axial (longitudinal) direction. The ducts of the endoscope are for example a therapeutic-instrument insertion duct also serving as a suction duct, an air-supply duct, and/or a water-supply duct.

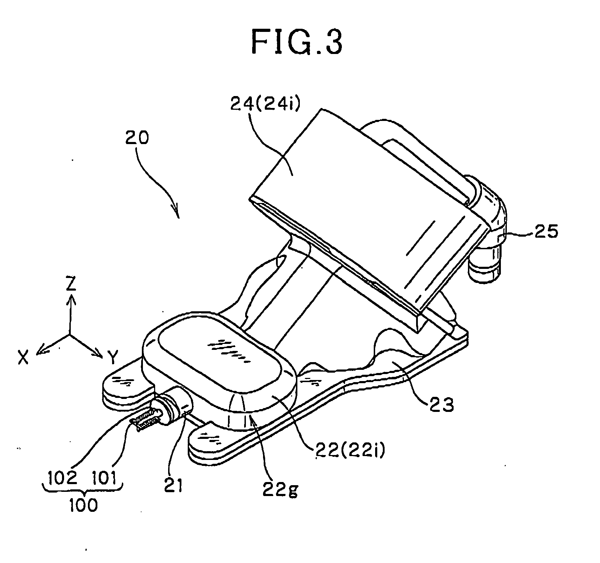

The brush cassette 20 is structured to supply fluid such as cleaning liquid and disinfecting liquid into the ducts such as therapeutic-instrument insertion duct and to accommodate therein a later-descried cleaning brush 100 (refer to Fig. 3). In the present embodiment, the brush cassette 20 is prepared as a disposable type of unit, but this is not a definitive list. Brush cassettes other than a disposable type are available as well. The structures of the brush cassette 20 and loading part 5 will be detailed later.

The top cover 3 extends to cover the brush cassette 20, when the cover is closed. At a portion of the top cover 3 which faces the brush cassette 20, there is formed a recessed cassette cover portion 3a. This cassette cover portion 3a has a back face portion 3h. In cases where the top cover 3 is closed toward the casing 2A (i.e., the main body unit 2), the back face portion 3h prevents the brush caste 20 loaded on the loading part 5 from touching the top cover 3. The top cover 3 is formed using a transparent or translucent material. Hence, after closing the top cover 3, an operator can visually observe both the cleaning bath 4 and the brush cassette 20 via the top cover 3.

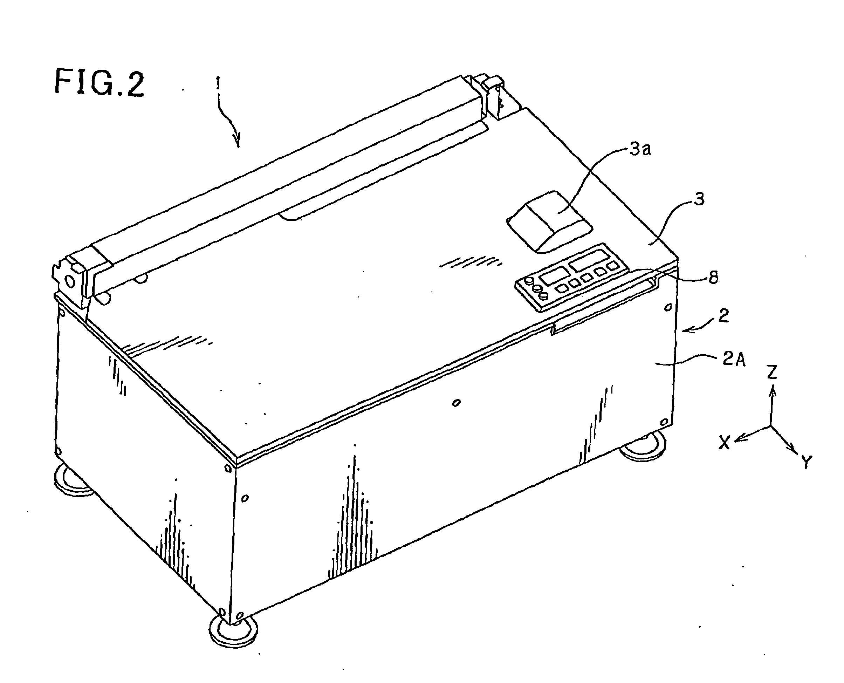

In addition, as shown in Fig. 2, an operation panel 8 is provided on the upper surface part of the top cover 3. This panel 8 is provided various operation and display devices which are interactive with operator's operations and massages to operators. Such operations and massages include start and stop operations for work, display for various steps (such as cleaning, rinsing, disinfecting, alcohol-flushing and dewatering steps), display of elapsed time, setting operation patterns of the various work steps.

In the endoscope washer-disinfector 1, the frame 2 accommodates therein various mechanisms which allow cleaning liquid, disinfecting liquid, rinsing water, alcohol, and air to selectively circulate into or through the cleaning bath 4 and cassette mounting portion 5 in the various work steps. Such fluids circulated are for cleaning and disinfecting the endoscope 50. For accomplishing the circulation, a tubing network with electromagnetic valves, check valves and other devices and various devices such as pumps and compressors are incorporated in the frame 2A. In addition, a control unit 110 (refer to Fig. 1) is also incorporated in the main body unit 2 and is configured to drive the foregoing devices in accordance with programs assigned to the various work steps.

The control unit 110 is formed as a computer equipped with memories and a CPU (central processing unit). The memories store therein programs for the work steps and a program for monitoring loaded states of a brush cassette 20 which will be detailed later. The CPU is configured to execute such programs. Depending on the actions of the CPU, the control unit 110 executes given processing.

The cleaning liquid, disinfecting liquid, and alcohol liquid are reserved in respective tanks placed in the frame 2A. Additionally, to this endoscope washer-disinfector 1, the rinsing water is supplied from the hydrant through a not-shown hose connected with the frame 2A. The tap water is used to dilute the cleaning liquid and disinfecting liquid.

Referring to Figs. 3-8, the brush cassette 20 being loaded to the loading part 5 will now be described.

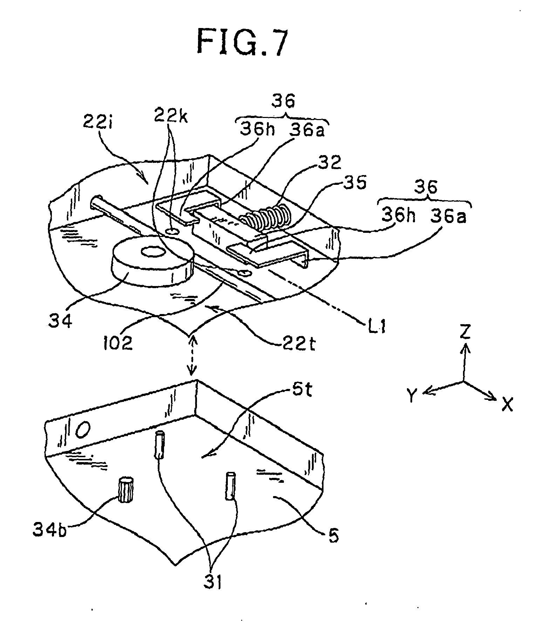

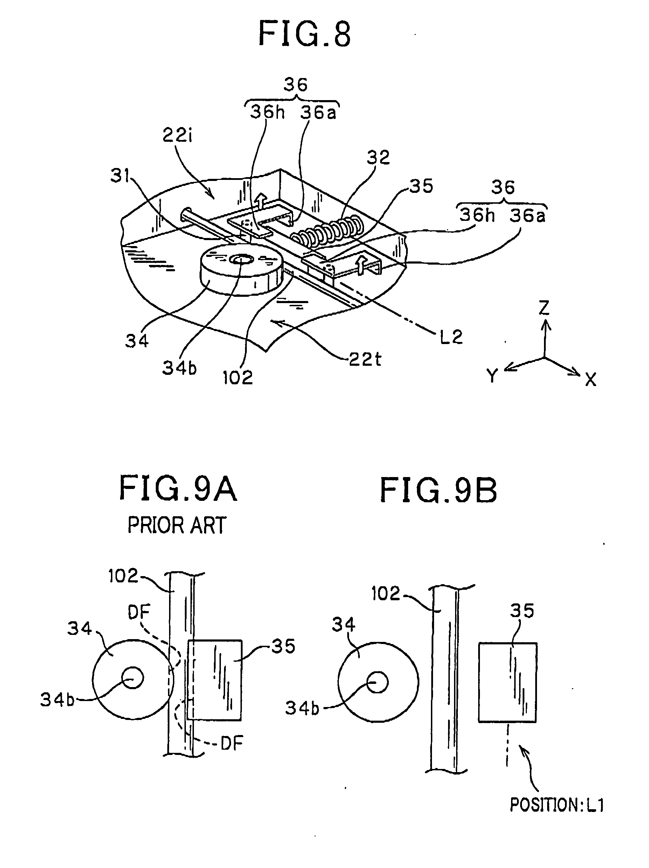

In Figs. 7 and 8, a guide member 35 and a fixing member 36, which will be detailed later, are pictorially shown, with their detailed appearances omitted for the sake of simplification.

As shown in Fig. 3, the brush cassette 20 is equipped with a connecting pipe 21 located on a side from which fluid is exhausted (hereinafter, referred to as an exhaust-side pipe), a roller accommodating portion 22, a base member 23, a brush accommodating portion 24, and a connecting pipe 25 located on a side from which the fluid is suctioned (hereinafter, referred to as a suction-side pipe).

Incidentally, the orthogonal coordinate system consisting of X-, Y-, and Z-axes will now be assigned to the brush cassette 20 shown in Fig. 3. In Fig. 3, the X-axis is set to the longitudinal direction of the exhaust-side pipe 2.

The exhaust-side pipe 21 is formed so that this pipe extends forth in the X-axis direction from an approximately central part of the roller accommodating portion 22 in the Y-axis direction.

When the loading part 5 is loaded with the brush cassette 20, the exhaust-side pipe 21 of this cassette 20 is positioned such that its distal end is directed to the cleaning bath 4 and opposed to a mouthpiece mounted at an end of a therapeutic-instrument insertion duct formed through the manipulating member 51 of the endoscope 50. At this time, the endoscope 50 is accommodated in the manipulating-member accommodating bath 6 of the cleaning bath 4.

Further, inside the exhaust-side pipe 21, there is formed a duc t (not shown) for allowing fluid such as cleaning liquid and the cleani ng brush 100 to pass therethrough.

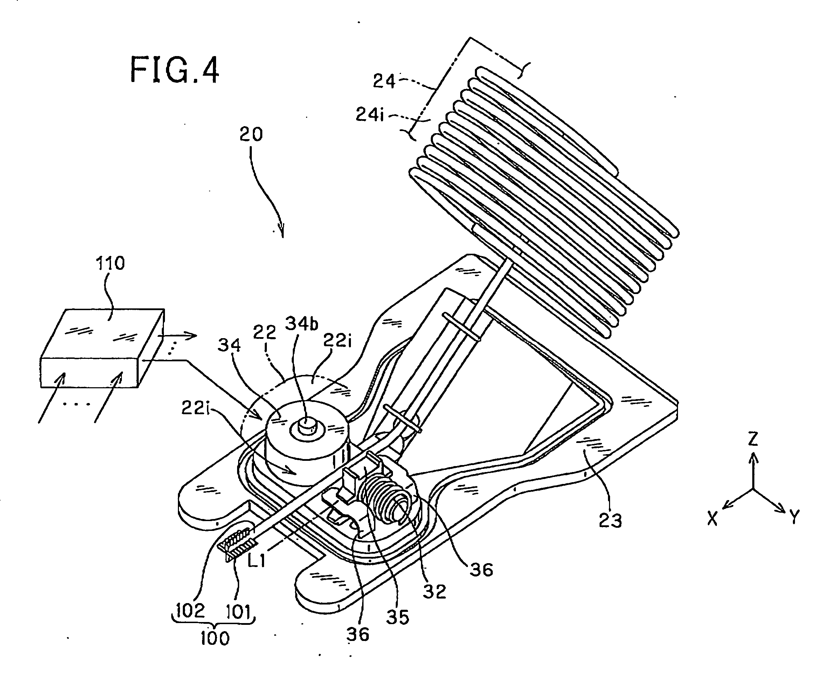

The roller accommodating portion 22 has a dome-shaped casing 22g composed of, at least partly, a transparent material or a semitransparent material and formed to have an approximately elliptical shape when viewed as a plan view. As shown in Fig. 4, this roller accommodating portion 22 has an inner space 22I communicating with the duct of the exhaust-side pipe 21 of the casing 22g. In this inner space 22i, there are equipped with a driving roller 34 serving as feeding and returning means, the guide member 35 serving as guide means, and the fixing members 36 (hereinafter, referred to as stoppers) serving as fixing means. The driving roller 34, guide member 35, and stoppers 36 are covered by the casing 22g.

In the present embodiment, the driving roller 34 has at least a surface portion made of rubber or the driving roller 34 is made of rubber as a whole. The guide member 35 also has a surface portion, made of rubber, which faces the driving roller 34. However, such a robber-made structure is just one example, and the driving roller 34 and the guide member 35 may have surface portions made of, for example, a resin or metal.

As a modification, the casing 22g may be transparent or semitransparent as a whole. As another modification, the casing 22g may have an observation window made of a transparent or semitransparent material.

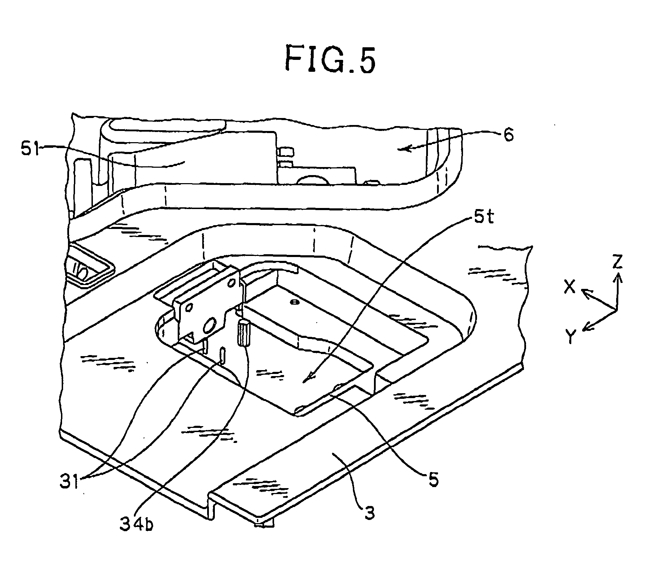

The driving roller 34 is able to couple with a roller shaft 34b and rotatable by the roller shaft 34b. The roller shaft 34b is built from a bottom portion 5t (refer to Fig. 5) of the loading part 5 and rotatable to the base portion 5t. The roller shaft 34b is rotated by an electric motor M, which is driven in response to control issued from a control unit 110.

The driving roller 34 is rotated together with either a forward rotation or a backward rotation of the roller shaft 34b. Hence, one of such rotations allows the cleaning brush 100 to be fed from a brush accommodating chamber 24i of the brush accommodating portion 24 to the therapeutic-instrument insertion duct. Further, the other rotation allows the cleaning brush 100 to be returned from the therapeutic-instrument insertion duct to the brush accommodating chamber 24i. Namely, the driving roller 34 enables the cleaning brush 100 to be fed and returned (i.e., advancement and retreat actions) between the brush accommodating chamber 24i and the duct.

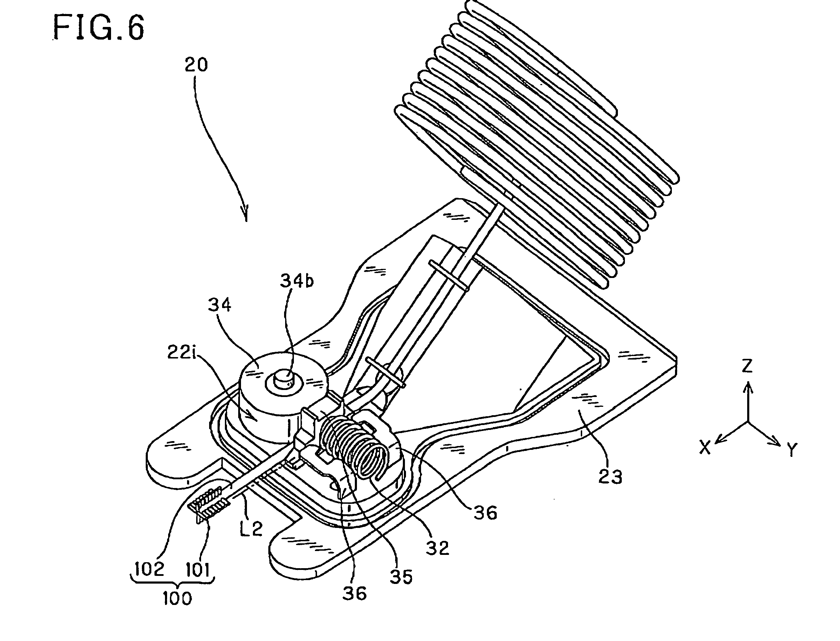

The guide member 35 is arranged movably from a separated position L1 (refer to Fig. 4) to a tight-holding position L2 (refer to Fig. 6) in the Y-axis direction. The separated position L1 is a position at which the guide member 35 is separated from the driving roller 34 by a predetermined distance. At this separated position L1, the shaft 102 (described later) of the cleaning brush 100 is free and loosened between the driving roller 34 and the guide member 35, with no pressure applied to the shaft 102.

In contrast, at the tight-holding position L2, the guide member 35 almost comes in contact with (i.e., located near) the driving roller 34 in a forcible manner to press the shaft 100 therebetween, thereby holding (pinching) the shaft 100 tightly. That is, at the tight-holding position L2, the guide member 35 applies pressure to pinch the cleaning brush 100 with the driving roller 34. This action enables the cleaning brush 100 to be not only guided to the driving roller 34 but also fed forward and pulled backward in collaboration with the driving roller 34. Moreover, the guide member 35 is linked with one end of a forcing spring 32 serving as forcing means. The other end of the forcing spring 32 is lined with the casing 22g. Hence the guide member 35 is constantly forced (pushed) by the forcing spring 32 toward the driving roller 34 in the Y-axis direction.

In cases where the brush cassette 20 is not loaded on the loading part 5, the guide member 35 is forced by the forcing spring 32 toward the driving roller 34 in the Y-axis direction. However, in this unloaded state, the stoppers 36 operate to fixedly locate (i.e., lock) the guide member 35 at the separated position L1 in the Y-axis dereliction (refer to Figs. 4 and 7).

The stoppers 36 are made up of, in the present embodiment, for example, two thin plate members. Specifically, as shown in Fig. 7, each of the two stoppers 36 has a stem portion 36a secured to a bottom 22t of the roller accommodating portion 22. This stem portion 36a connects the bottom 22t and an arm portion 36h of each stopper 36. The arm portion 36h extends from the stem portion 36a along a side of the guide member 35 in the Y-axis direction and has a distal portion bent inward (in the X-axis diction) to partly surround the guide member 35. Thus, these two stoppers 36 are able to block the guide member 35 from moving responsively to the forcing spring 32 toward the driving member 34 in the Y-axis diction, if the respective arm portions 36h are lower in height in the Z-axis direction than the guide member 35. Thus the guide member 35 is fixedly located, i.e., positionally locked, at the separated position L1.

Further, through the bottom 22t of the roller accommodating portion 22, through-holes 22k are formed to allow the roller shaft 34b and later-described two unlocking pins (or shafts) 31 to pass through, respectively (refer to Fig. 7). It should be mentioned that Fig. 7 shows only the two through-holes 22k to be opposed to the two unlocking pins 31. The through-holes 22k are located in the X-Y plane such that those through-holes 22k are positionally in accord with the arm portions. 36h of the two stoppers 36 as well as the center of the driving roller 34.

The base member 23 is a member directly contacting the bottom portion 5t of the loading part 5, when the brush cassette 20 is loaded (mounted) to the loading part 5. Although not shown, on the base member 23, there is formed a duct which communicates with the inner chamber 22i of the roller accommodating portion 22 and which allows the cleaning brush 100 and liquids such as cleaning liquid to pass therethrough.

The brush accommodating portion 24 is a cylindrical member with a short axial length, which is secured on the base member 23 to have a tilt to the backward direction in the X-axial direction. Inside the brush accommodating portion 24, a brush accommodating chamber 24i is formed. Before feeding and after pulling back the shaft 102 of the cleaning brush 100, the chamber 24i accommodates the shaft 102 in its wound attitude. Though not shown in detail, the brush accommodating chamber 24i is formed to communicate with a duct (not shown) of the base member 23, an inner chamber 22i of the roller accommodating portion 22, and the exhaust-side pipe 21.

The suction-side pipe 25 is located to extend downward in the Z-axis direction and has one end connected with a rear end portion of the brush accommodating portion 24 in the X-axis direction. The suction-side pipe 25 communicates with the brush accommodating chamber 24i, the duct of the base member 23, the inner chamber 22i of the roller accommodating portion 22, and the exhaust-side pipe 21.

The suction-side pipe 25 is a member for introducing fluid such as cleaning liquid and disinfecting liquid into the brush accommodating chamber 24i. The introduced cleaning and disinfecting liquids are used to clean and disinfect the cleaning brush 100. The liquids introduced into the chamber 241 flow in turns through the duct of the base member 23, the inner chamber 22i of the roller accommodating portion 22, and the exhaust-side pipe 21, and finally enter, for example, therapeutic-instrument insertion duct of the endoscope 50 for cleaning and disinfection.

As shown in Fig. 4, the cleaning brush 100 is equipped with a brush portion 101 and the foregoing thin and long shaft 102 having a distal end at which the brush portion 101 is secured. Utilizing the cooperation between the driving and guide rollers 34 and 35, the cleaning brush 100 is formed to advance, from the brush accommodating chamber 24i, in turns, through the duct (not shown) of the base member 23, the inner chamber 22i of the roller accommodating portion 22, and the exhaust-side pipe 21, and is guided into, for example, the therapeutic-instrument insertion duct of the endoscope 50.

Then the brush portion 101 of the cleaning brush 100, which has been inserted into the therapeutic-instrument insertion duct, is made to advance and retreat along the duct, depending on the forward and backward rotations of the driving roller 34. The brush portion 101 is composed of lots of hairs planted at the distal end of the shaft 102. The hairs extend in the radial direction of the shaft 102. Hence, the advancing and retreating actions of the cleaning brush 100, that is, the shaft 102 permit the brush portion 101 to brush-clean the inside of the therapeutic-instrument insertion duct with the aid of the cleaning liquid.

The brush cassette 20, which is configured as above, can be loaded to the loading part 5 of the frame 2A of the endoscope water disinfector 1.

Meanwhile the loading part 5 has the bottom portion 5t, which has been stated, on which the base member 23 of the brush cassette 20 is mounted.

As shown in Figs. 5 and 7, in the bottom portion 5t, the foregoing roller shaft 34b and the two unlocking pins 31 are placed to stand up in the Z-axis direction.

When the brush cassette 20 Is loaded to the loading part 5, the roller shaft 34b is made to penetrate one of the through-holes 22 of the bottom 22t of the roller accommodating portion 22, and then to be fit with the driving roller 34. Thus the driving roller 34 is rotatable by the roller shaft 34b.

The unlocking pins 31 compose part of the unlocking means for releasing (unlocking) the fixed (locked) state of the guide member 35, as described. Specifically, when the brush cassette 20 is loaded to the loading part 5, the two unlocking pins 31 operate to release the positionally fixed state of the guide member 35 at the separated position L1, which has been locked so far by the two stoppers 36. As a result, the guide member 35 is forced by the forcing spring 32, so that the guide member 35 is forcibly moved to the tight-holding position L2 in the Y-axis direction.

More specifically, when the brush cassette 20 is loaded to the loading part 5, the two unlocking pints 31 are made to pass through two of the through-holes 22k of the bottom 22t of the roller accommodating portion 22. Hence, as shown in Fig. 8, these unlocking pins 31 forcibly lift up the arm portions 36h of the stoppers 36, respectively, in such a manner that the arm portions 36h are higher than the guide member 35 in the Z-axis direction. These lifting actions release the guide member 35 which has been positionally fixed (locked) by the two arm portions 36h, resulting in that the guide member 35 is adequately pressed to the driving roller 34 by the forcing spring 32. Hence the guide member 35 almost contacts the driving roller 34, thereby positioning at the tight-holding position L2.

The operations and advantages in the present embodiment will now be described.

First of all, an endoscope 50 being cleaned and disinfected is mounted in the cleaning bath 4 of the endoscope washer and disinfector 1. Concretely, in the manipulating-member accommodating bath 6, the manipulating member 51 of the endoscope 50 is accommodated, and in the insertion-tube accommodating bath 7, the insertion tube 52 is accommodated in its wound attitude.

A brush cassette 20 which has not been in use is then loaded to the loading part 5. Before loading the cassette 20, as shown in Figs. 4 and 7, the guide member 35 is located at the separated position L1, where the guide member 35 is separated from the driving member 34 in the Y-axis direction. In this location, the two stoppers 36 lock the guide member 35 by the arm portions 36h from moving in the Y-axis direction.

Incidentally, the brush cassette 20 is manufactured such that the two stoppers 36 realize the separation of the guide member 35 from the driving roller 34 and the poisoning thereof at the separated position L1.

The inside of the roller accommodating portion 22 can be seen through the transparent or semitransparent casing 22g. For this reason, the operator is able to visually confirm that the brush cassette 20 has not been in use yet in an easier manner by observing the guide member 35 located at the separated position L1.

As shown in Figs. 6 and 8, when the brush cassette 20 is loaded to the loading part 5, the roller shaft 34b passes through the one through-hole 22k of the bottom 22t of the roller accommodating portion 22 on the way to completion of the loading. Thus the roller shaft 34b is fit with the driving roller 34 for the rotatable support. Concurrently with this action, the two unlocking pints 31 pass through the remaining two through-holes 22k so as to lift up the arm positions 36h of the stoppers 36 higher than the guide member 35 in the Z-axis direction. As described, these lifting actions release (unlock) the positionally fixed (locked) guide member 35.

Responsively to this unlocking action, the guide member 35 is forcibly pushed toward the driving roller 34 in the Y-axis direction, thus being located at the tight-holding position L2. Accordingly, the shaft 102 of the cleaning brush 100 is held tight (pinched) between the driving roller 34 and the guide member 35 at a preset holding pressure.

Then the driving roller 34 is driven to rotate in a predetermined direction. At this time, the shaft 102 is pushed toward the driving roller 34 by the guide member 35. Namely, the guide member 35 presses and holds the shaft 102 to and with the driving roller 34, so that the shaft 102 is guided to advance and retreat. Hence rotating the driving roller 34 enables the cleaning brush 100 to go forward from the brush accommodating chamber 24i to, for example, the therapeutic-instrument insertion duct of the endoscope 50 via the exhaust-side pipe 21, together with the cleaning liquid. In this state, the forcing spring 32 keeps pushing the guide member 35 at the preset constant pressure. Hence the shaft 102 can be subjected to its advancing and retreating motions at the constant pressure.

In response to the advancement, the cleaning brush 100 is inserted into the therapeutic-instrument insertion duct. After this, the driving roller 34 is driven to rotate depending on the rotating directions of the electric motor M. The rotations of the driving roller 34 make it possible that the cleaning brush 100 is subjected to the back-and-forth motions repeated along the duct by a predetermined number of times. Thus the brush portion 101 is able to brush and clean the therapeutic-instrument insertion duct using the cleaning liquid.

After finishing cleaning the duct, the rotation of the driving roller 34, which responds to the rotation of the electric motor M in the predetermined direction, causes the cleaning brush 100 to be pulled from the therapeutic-instrument insertion duct. The pulled cleaning brush 100 is then returned into the brush accommodating chamber 24i via the exhaust-side pipe 21, before the brush cassette 20 is unloaded form the loading part 5.

This unloading action accompanies removal of the two unlocking pins 31 such that the pins disappear from the inner chamber 22i of the roller accommodating portion 22 through the two through-holes 22k. However, the two stoppers 36 are still kept higher than the guide member 35 in the Z-axis direction. The reason is that the guide member 35 is kept being pushed to the driving roller 34 by the forcing spring 32 in the Y-axis direction, even after the two unlocking pins 31 disappear. That is, the guide member 35 will never return to the separated position L1, keeping the tight-holding position L2.

When unloaded from the loading part 5, the cleaning brush 100 is entirely retuned and accommodated to and in the brush accommodating chamber 24i. Thus, the shaft 102 is no longer pinched between the guide member 35 and the driving roller 34. As a result, the appearance of the brush cassette 20 differs between before use and after use. In the case of being used once, it is impossible to see the brush portion 101 inside or outside the exhaust-side pipe 21. It is therefore possible to determine whether the cassette is before use or after use by seeing the appearance of bush cassettes.

In this way, in the present embodiment, when the brush cassette 20 is unloaded on the loading part 5, i.e., is not in use, the guide member 35 is located at the separated position L1 apart from the driving roller 34 by the stoppers 36. Hence, in this case, the shaft 102 will not be pressed between the guide member 35 and the driving roller 34. Unlike the conventional, it is therefore possible to avoid at least one of the driving roller 34 and the guide member 35 from deformed fixedly.

Figs. 9A and 9B exemplify how such a deformation is prevented. Both of Figs. 9A and 9B show Z-axial pictorial views of the driving roller 34 and the guide member 35. If the shaft 102 is held tight between both members 34 and 35 for a long time, the driving roller 34 and/or the guide members 35 may have a recess (deformation) in accord with the shape of the shaft 102, causing non-flexible deformations DF thereon, as shown in Fig. 9A. By contrast, in the present embodiment, a brush cassette 20 which has not been in use allows its guide member 35 to be located at the separated position L1 as shown in Fig. 9B, so that no deformation is caused.

In consequence, the brush cassette 20 which is not in use is subjected to its long-term storage and/or transportation, it cannot be found such deformations when the cassette is actually used for cleaning and disinfection. The brush cassette 20 according to the present embodiment has no such deformations in most cases, whereby the cleaning brush can be moved back and forth smoothly. Accordingly, the cleaning performance cannot be lessened and the cleaning and disinfecting work cannot be reduced in efficiency.

On the contrary, the brush cassette can be stored and transported longer than the conventional. Further, even if environment conditions of temperature and humidity are harder, deformations are unlikely to occur, because there is no pressure due to the shaft.

Further, in the present embodiment, loading the brush cassette to the main body unit automatically releases the guide member from being fixed. Then the cleaning brush is held tight between the driving roller and the guide member, so that the cleaning brush can be fed into and pulled from each duct of an endoscope reliably, even after its long-term transportation and storage.

Meanwhile, an operator can see the inside of the chamber 22i of the roller accommodating portion 22 through the casing 22g. In other words, the operator visually observes whether or not the guide member 35 is present at the separated position L1 with ease. Hence the operator can recognize whether a brush cassette 20 is new or not. When the guide member 35 is located at the separated position L1, the cassette has not been in use.

Moreover, even when the brush cassette 20 is unloaded from the loading part 5, the guide member 35 is kept at the tight-holding position L2, thus no returning to the separated position L1. An operator can see the current position of the guide member 35, as described above, to judge whether or not the brush cassette 20 has been used once. In the present embodiment, reuse of a brush caste is unavailable. Because brush cassettes can easily be judged as to whether they have already been used once, it is possible to avoid erroneous reuse.

In addition, even if a brush cassette which has already been used once is tried to be loaded, there is no operational feeling which can be obtained in the normal loading (a feeling caused when the guide member is moved with resistance). This is because the guide member 35 is already forced to be located at the tight-holding position L2. Thus this different operational feeling is also helpful in preventing brush cassette from being loaded erroneously.

### (Second embodiment)

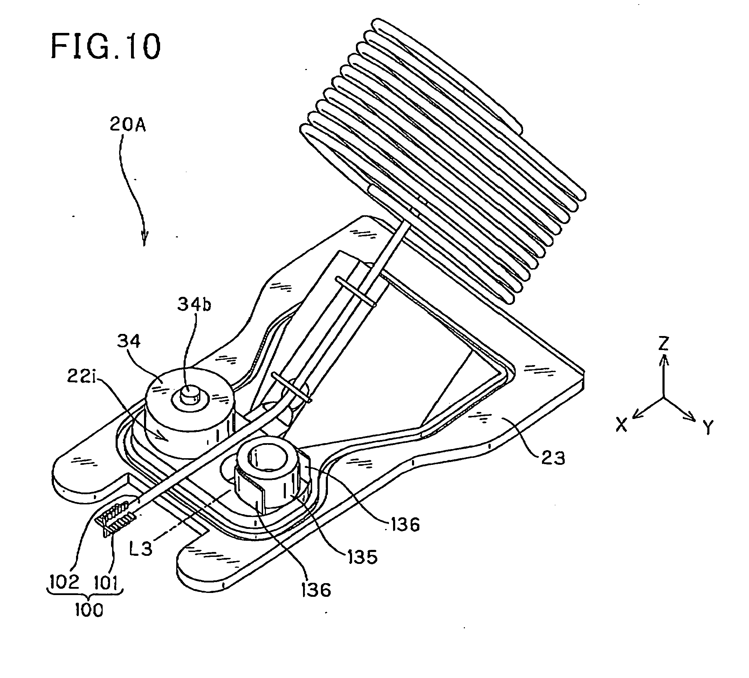

Referring to Figs. 10-17, an endoscope washer-disinfector according to the second embodiment will now be described.

The endoscope washer-disinfector according to the second embodiment differs from that described in the first embodiment in the shapes of the guide member and stoppers of the brush cassette and the shapes of the unlocking pines disposed at the loading part. Additionally, the unlocking pins themselves do not move the stoppers, but move the guide member, which is another different structure from that of the first embodiment. In the followings, the description will be given mainly to such differences. And, in the present embodiment, the same or identical components to those in the first embodiment will be given the same reference numerals as those in the first embodiment.

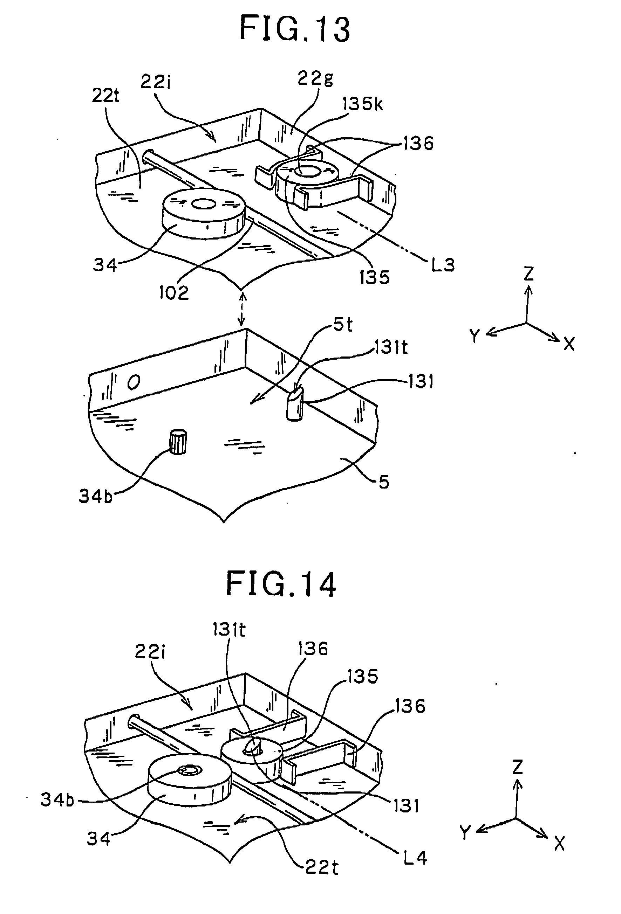

Incidentally, in Figs. 13 and 14 later described, a guide member 135 and stoppers 136 are pictorially depicted, with their detailed structures omitted from their depiction.

Fig. 10 partially shows a brush cassette 20A adopted by an endoscope washer and disinfector according to the second embodiment. As shown therein, the brush cassette 20A has a roller accommodating portion 22 whose casing 22g has an inner chamber 22i. In this chamber 22i, there are provided a driving roller 34, a guide member 135 serving as guide means, and stoppers 136 serving as fixing means. The driving roller 34, guide member 135, and stopper 136 are covered by the casing 22g, described as before.

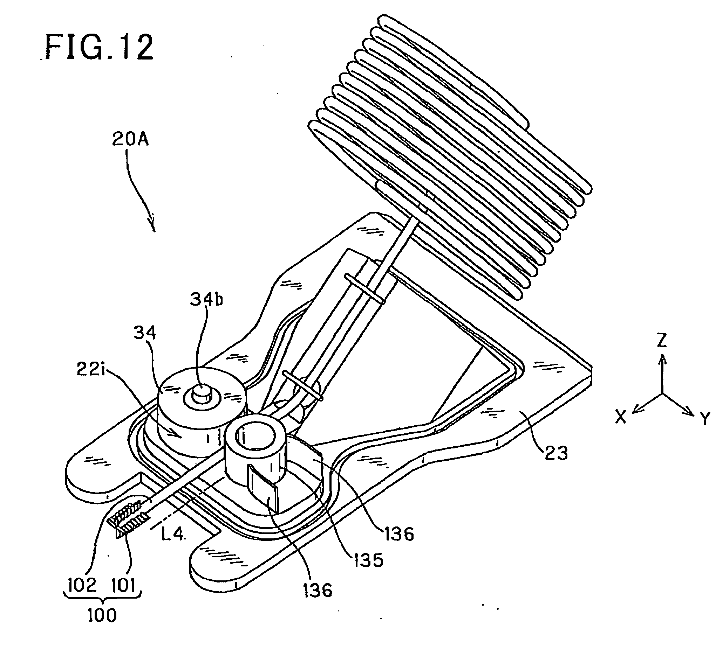

The guide member 135, which is for example made of rubber, is disposed to be movable in the Y-axis direction. Concretely, the guide member 135 is disposed to hold a cleaning brush 100 tightly with the driving roller 34 at a tight-holding position L4 as shown in Figs. 12 and 14. This allows a cleaning brush 100 to be held (guided) between the driving roller 34 and the guide member 135 and to go back and forth selectively by rotating the driving roller 34.

The guide member 135 is formed into, for example, a rubber-made cylindrical roller having a short axis, and is build in the Z-axis direction in a condition where the member 135 is movable in the Y-axis direction. At the central part of the guide member 135, a through-hole 135k is formed in its axial direction.

The stoppers 136 are members to fix the guide member 135 at a separated position L3 (refer to Figs. 10 and 13) which is apart from the driving roller 34 in the Y-axis direction, when the brush cassette 20A is not loaded to the loading part 5 (i.e., the cassette is not in use). The stoppers 136 are two members each having a spring force and being opposed to each other in the X-axis direction.

Specifically, as shown in Fig. 13, the two stoppers 136 are placed on the bottom 22t of the roller accommodating portion 22 so that the stoppers are opposed to each other in the X-axis direction. One end of each of the stoppers 136 are made free, while the other end thereof is fixed to the wall of the bottom 22t. The stoppers 136 hold tightly the guide member 135 in the X-axis direction to fix it at the separated position L3.

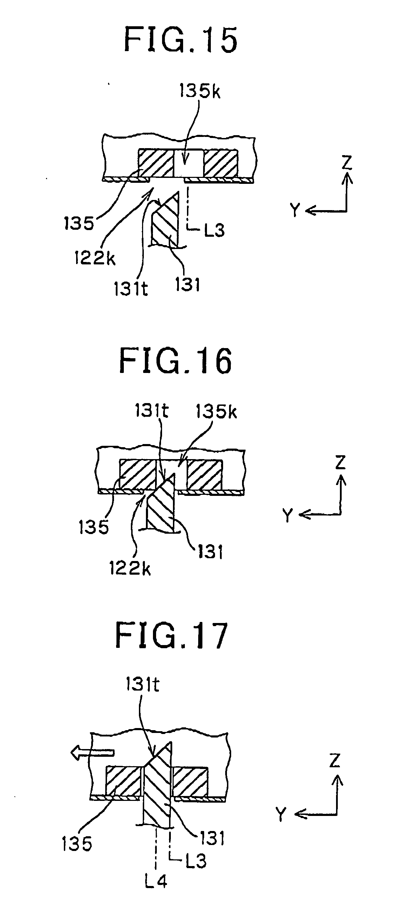

In addition, in the bottom 22t of the roller accommodating portion 22, two through-holes 122k are formed at positions which are allowed to face the through-hole 135k of the guide member 135 and the driving roller 34 (refer to Figs. 15 and 16). The roller shaft 34b and a later-described unlocking pin 131 are able to pass through these through-holes 122k, respectively. Incidentally, Figs. 15 and 16 show only the through-hole 122k facing the through-hole 135k.

The brush cassette 20A thus constructed is detachably loaded to the loading part 5 of the frame 2A of the main body unit 2.

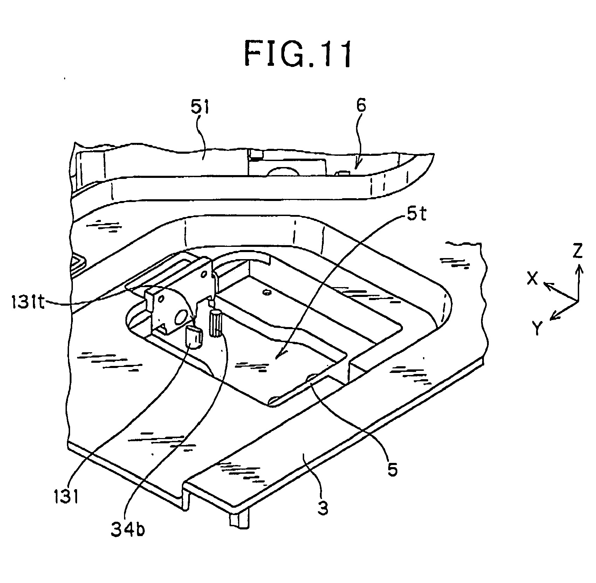

On the other hand, as shown in Figs. 11 and 13, on the bottom portion 5t of the loading part 5, the roller shaft 34b and the unlocking pin 131 (serving as unlocking means) are fixedly placed so that the shaft and the pin are both built therefrom in the Z-axis direction. In the Y-axis direction, as shown in Fig. 15, the unlocking pin 131 is located nearer to the driving roller 34 than the through-hole 135k of the guide member 135 which is movable.

The unlocking pin 131 is used to release (unlock) a locked state of the guide member 135 at the separated position L3 by the stoppers 136, in response to loading the brush cassette 20A to the loading part 5. This allows the guide member 135 to move to the tight-holding position L4 in the Y-axis direction.

More precisely, the unlocking pin 131 has a top on which a tapered surface 131t is formed, as shown in Figs. 11, 14 and 15. The tapered surface 131t has a predetermined tilt to the Y-axis direction. This unlocking pin 131 is able to provide an unlocking function during a process in which the brush cassette 20A is loaded to the loading part 5. As shown in Fig. 16, the unlocking pin 131 passes through one through-hole 122k of the bottom 22t of the roller accommodating portion 22, and then the top of the unlocking pin 131 is inserted into the through-hole 135k. As the unlocking pin 131 is inserted deeper as shown in Fig. 17, the top thereof, that is, the tapered surface 131t, creates a component of force acting on the guide member 135 in the Y-axis direction. This force component enables the guide member 135 to forcibly be released from the stoppers 136, whereby the guide member 135 is pushed toward the driving roller 34 in the Y-axis direction.

As a result, when the brush caste 20A has been loaded to the loading part 5 completely, the unlocking pin 131 also accomplishes a complete insertion into the through-hole 135k of the guide member 135, as shown in Fig. 17. This completion of the insertion finalizes the unlocked action of the guide member 135 which has been locked so far by the stoppers 136.

The operations and advantages according to the present embodiment will now be described, in which the same or equivalent operations and advantages as or from those in the first embodiment will now be omitted for simplicity.

First, an endoscope 50 to be cleaned is accommodated into the cleaning bath 4 of the main body unit 2, and then the brush cassette 20A is loaded to the loading part 5.

If the brush cassette 20A is not in use, i.e., new one, the two stoppers 136 hold tightly the guide member 135 at the separated position L3 (locked state) in the inner chamber 22i of the roller accommodating portion 22. Thus the guide member 135 is apart from the driving roller 34. This separated state can be confirmed visually by the operator through the casing 22g, because it is transparent or semitransparent.

Then, loading the brush cassette 20A to the loading part 5 begins. As the loading action advances, the roller shaft 34b is gradually inserted into the driving roller 34 via one of the through-holes 122 of the bottom 22t of the roller accommodating portion 22. Concurrently, as shown in Fig. 16, the unlocking pin 131 is also gradually inserted into the through-hole 135k of the guide member 135 through the other through-hole 122k of the bottom 22t. With an advancement in the inserting action, the guide member 135 is unlocked and pushed away toward the driving roller 34 in the Y-axis direction, as described before.

When the loading of the brush cassette 20A has been completed, the driving roller 34 is fitted with the roller shaft 34b, as shown in Figs. 12 and 14. In addition, at this time, the guide member 135 is completely unlocked by the unlocking pin 131, as shown in Fig. 17.

As a result, as shown in Fig. 14, the guide member 135 is forcibly pushed toward the driving roller 34 in the Y-axis direction, with the ends of the stoppers 136 still touching the guide member 135. Thus, the guide member 135 is located at the tight-holding position L4, resulting in that the shaft 102 of the cleaning brush 100 is held tight between the guide member 135 and the driving roller 34.

Then, in the same manner as that in the first embodiment, the cleaning and disinfecting operations are carried out. After those operations, the cleaning brush 100 is pulled back from the duct of the endoscope 50, and then pulled into the brush accommodating chamber 24i by rotating the driving roller 34. The brush cassette 20A is then unloaded from the loading part 5.

In the unloading step, the unlocking pint 131 disappears from the inner chamber 22i of the roller accommodating portion 22 via the through-hole 122. However, the distal ends of both stoppers 136 still keep touching the guide member 135, with continuously pushing the guide member 135 toward the driving roller 34 in the Y-axis direction. That is, even after removing the unlocking pin 131 from the guide member 135, the guide member 135 is subjected to continuous pushing to the driving roller 35 by the stoppers 136. Thus the guide member 135 will not return to the separated position L3, keeping the tight-holding position L4 (i.e., locked thereat).

In addition, after the cleaning, the cleaning brush 100 is pulled back to the brush accommodating chamber 24i for accommodation. Hence, the shaft 102 is no longer located between the guide member 135 and the driving roller 34.

The foregoing configurations according to the second embodiment provide the similar advantages to those in the first embodiment. In particular, in the brush cassette 20A after the cleaning, the guide member 135 is locked at the tight-holding position L4. This prevents reuse of the cassette, even if an operator tries to reuse it. Thus it is prevented that a used brush cassette is again loaded improperly. Further, compared to the first embodiment, a mechanism for moving the guide member 135 to the tight-holding position L4 is simplified.

### (Third embodiment)

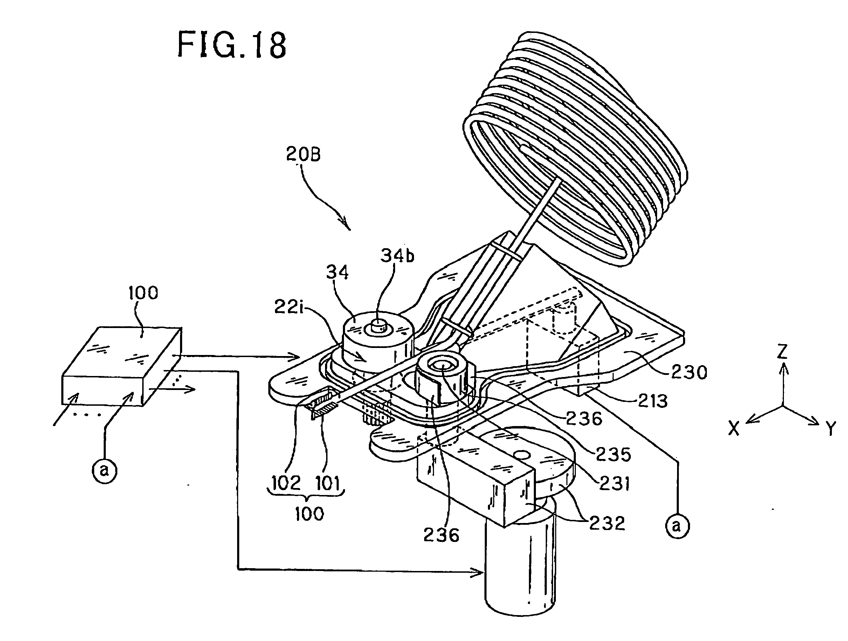

Referring to Figs. 18-19, an endoscope washer-disinfector according to a third embodiment of the present invention will now be described.

The endoscope washer-disinfector according to the third embodiment differs from that of the second embodiment in the shape and mechanism of the unlocking pin. In the present embodiment, the same or identical components to those in the second embodiment will be given the same reference numerals as those in the second embodiment.

Fig. 18 shows part of a brush cassette 208 adopted by an endoscope washer-disinfector according to this third embodiment. As shown in Fig. 18, the brush cassette 20B has a roller accommodating portion 22 in which an inner chamber 22i is formed by a casing 22g. In the chamber 22i, a driving roller 34, a guide member 235 serving as guide means, and stoppers 236 serving as locking (fixing) means are mounted.

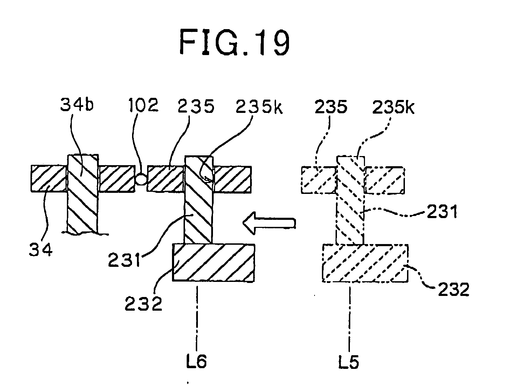

The guide member 235 is disposed to tightly hold the cleaning brush 100 with the driving roller 34 at the tight-holding position L6 shown in Fig. 19. This makes it possible that the cleaning brush 100 is held (guided) to the driving roll 34 and allowed to go back and forth selectively by rotating the driving roller 34.

The guide member 235 is formed into, for example, a rubber-made cylindrical roller having a short axis, and is build in the Z-axis direction in a condition where the member 235 is movable in the Y-axis direction. At the central part of the guide member 235, a through-hole 235k is formed in its axial direction (refer to Fig. 19).

The stoppers 236 are members to lock (i.e., fix) the guide member 235 at a separated position L5 which is apart from the driving roller 34 in the Y-axis direction, when the brush cassette 20B is not loaded to the loading part 5 (i.e., the cassette is not in use). The stoppers 236 are formed similarly to those in the second embodiment. Namely, as shown in Fig. 18, the stoppers 136 are two members each being located to extent along the Y-axis direction, having a spring force, and being opposed to each other in the X-axis direction.

Specifically, as shown in Fig. 18, the two stoppers 236 are placed on the bottom 22t of the roller accommodating portion 22 so that the stoppers are opposed to each other in the X-axis direction. One end of each stopper 236 is fixed. The stoppers 236 hold tightly the guide member 235 in the X-axis direction to lock (i.e., fix) it at the separated position L5.

In addition, in the bottom 22t of the roller accommodating portion 22, two through-holes (not shown) are formed at positions which are allowed to face the through-hole 235k of the guide member 235 and the driving roller 34. The roller shaft 34b and a later-described unlocking pin 231 are able to pass through these through-holes, respectively.

The brush cassette 20B thus constructed is detachably loaded to the loading part 5 of the frame 2A of the main body unit 2.

On the other hand, as shown in Fig. 18, on the bottom portion 5t of the loading part 5, the roller shaft 34b, the unlocking pin 231 (serving as unlocking means), and a moving mechanism 232 are arranged. The moving mechanism 232 functions as moving means for moving the unlocking pin 231 between the separated position L5 and the tight-holding position L6. Additionally, the moving mechanism 232 is of electric-driven type and its motions are controlled by the control unit 110 placed in the main body unit 2.

In the loading part 5, a sensor 213 for sensing loading and unloading actions of the brush cassette 20B is arranged. This sensor 213 is selected from appropriate sensing means including proximity sensors, such as a switch, optical sensor, magnetic sensor, or ultrasound sensor.

After loading the brush cassette 20B onto the loading part 5, the guide member 235 is, in a fitted manner, inserted into the through-hole 235k of the guide member 235. Then the unlocking pin 231 is moved by the moving mechanism from the separated position L5 to the tight-holding position L6 in the Y-axis direction. This enables the two stoppers 236 to release (unlock) the guide member 235.

The sensor 213 senses the loading action of the brush cassette 20B to the loading part 5, and responsively to this sensing, a sensing signal is supplied to the control unit 110. In reply to the sensing signal, the control unit 110 outputs a driving signal to drive the moving mechanism 232. Hence the moving mechanism 232 allows the guide member 235 to be released (unlocked) from its fixed (locked) state at the separated potion L5, by moving the unlocking pin 231, thus moving the guide member 235 to the tight-holding position L6.

Meanwhile, upon sensing the unloading action of the brush cassette 20B, the sensor 213 also supplies a corresponding signal to the control unit 110. The control unit 110 answers this signal and controls the drive of the moving mechanism 232 to move the unlocking pin 231 from the tight-holding position L6 to the separated initial position L5 for the next loading of another new brush cassette.

The moving mechanism 232 is formed as a mechanism that us es an electric motor and gears or rollers in a combined manner or a solenoid mechanism.

The operations and advantages of the third embodiment will now be described. The same or similar operations and advantages as and to those in the second embodiment will be omitted here.

An endoscope 50 being cleaned is first accommodated in the cleaning bath 4. Then the brush cassette, which is new one, is loaded onto the loading part 5. This loading action is sensed by the sensor 213.

Before the brush cassette 20B is not been loaded, the two stoppers 236 tightly hold the guide member 235 therebetween in the inner chamber 22i of the roller accommodating portion 22. Thus the guide member 235 is located at the separated position L5 (refer to Fig. 19). This location of the guide member 235 can be visually confirmed by the operator in the same manner explained before.

In response to loading the brush cassette 20B to the loading part . 5, the roller shaft 34b is fitted with the driving roller 34 via one through-hole 122k. Concurrently with this action, the unlocking pin 231 is inserted into the through-hole 235k of the guide member 235 (refer to Fig. 19)

Then the driving signal from the control unit 110 activate the moving mechanism 232, whereby as shown in Fig. 19, the unlocking pin 231 is obliged to move from the separated position L5 to the tight-holding position L6. This movement allows the guide member 235 to be released (unlocked) from the two stoppers 236.

Accordingly, the guide member 235 is pushed to come into contact with driving roller 34 at the tight-holding position L6, thereby tight-holding the shaft 102 of the cleaning brush 100 between both members 235 and 34.

After this, similarly to that in the foregoing embodiments, the cleaning brush 100 is used to clean the duct of the endoscope 50. Then the control unit 110 operates to control the rotation of the driving roller 34 so that the cleaning brush 100 is pulled back to the brush accompanying chamber 24i from the duct.

The operator then removes the used brush cassette 20B from the loading part 5, thereby making the unlocking pin 231 disappear from the inner chamber 22i of the roller accommodating portion 22 via the through-hole 122k.

This removal of the cassette 20B is sensed by the sensor 213. In reply to this sensing, the control unit 110 controls the unlocking pin 231 so that this pin returns to its initial separated position L5, before ending the movement control of the guide member 235.

Incidentally, in the same as that in the second embodiment, the free distal ends of the stoppers 236 keep touching the guide member 235 so as to put toward the driving roller 34, even after loading the brush cassette 20B. That is, with the unlocking pin 231 removed, the guide member 235 is continuously pushed toward the driving roller 34 by the stoppers 236. The guide member 235 will not return to the separated position L5, being locked at the tight-holding position L6.

In this state, the cleaning brush 100 is totally pulled in the brush accommodating chamber 24i, with no shaft between the guide member 135 and the driving roller 34.

Therefore it is possible to enjoy the similar advantages to those provided in the second embodiment.

A modification of the third embodiment will now be explained.

In the foregoing third embodiment, the unlocking pin 231 is arranged to the moving mechanism 232 incorporated in the loading part 5. However, this is not a definitive list. A further example is to arrange an unlocking pin, which Is similar to the foregoing one 231, to the brush cassette 20B. In this arrangement, the unlocking pin is placed to extend downward in the Z-axis direction from the guide member 235 or the stopper 236.

In this case, the unlocking pin is fitted with a reception recess formed at the moving mechanism 232, when this cassette is loaded to the loading part 5. Then, in reply to drive of the moving mechanism 232, the unlocking pin 231, i.e., the guide member 235, is moved from the separated position L5 to the tight-holding position L6 in the Y-axis direction.

Thus, in such a modified cassette, the locked state of the guide member 235 at the separated position L5 can be unlocked by moving the unlocking pin 231, whereby the similar advantages to those in the third embodiment can be gained.

By the way, in the foregoing first to third embodiments, the duct(s) of the endoscope being cleaned will not be limited to the therapeutic-instrument insertion duct, but may be a water-supply duct, an air-supply duct, or a forward water-supply duct. Furthermore, in the endoscope water and disinfector according to the first to third embodiments, cleaning of ducts can be performed alone using the brush 100, thus providing the functions as an endoscope cleaning apparatus. Of course, the present invention may be reduced into practice as an endoscope cleaning apparatus dedicated to only cleaning of the ducts of the endoscope.

Although the description above contains many specificities, these should not be construed as limiting the scope of the invention but as merely providing illustrations of some of the presently preferred embodiments of the present invention. Thus the scope of the present invention should be determined by the appended claims.

## Claims

1. An endoscope washer-disinfector for, at least, cleaning and disinfecting an endoscope with a duct, comprising:
a frame having a bath used for cleaning and disinfection of the endoscope accommodated in the bath;
a brush cassette detachably loaded to a loading part of the frame and formed to comprise
a brush element cleaning the duct of the endoscope,
a shaft connected with the brush element;
a first member and a second member formed to hold the shaft tight therebetween and to make the tight held shaft advance and retreat to and from the duct, and
a lock device i) separating the second member from the first member so that the second member is located at a first position and ii) locking the separated second member, when the brush cassette is not loaded on the loading part, while iii) unlocking the locked second member in response to an operating force given responsively to a loading action given to the brush cassette and iv) allowing the second member to be moved to a second position where the first and second members hold tight the shaft therebetween, when the brush cassette is loaded to the loading part; and
an unlocking commander unlocking the locked second member by providing the operating force to the lock device when the brush cassette is loaded to the loading part.

2. The endoscope washer-disinfector according to claim 1, wherein the lock device comprises forcing means forcing the second member so as to cause the second member to move from the first position to the second position along a predetermined direction intersecting the shaft, and prohibiting means prohibiting the second member from being forcibly moved by the forcing means,
wherein the unlocking commander comprises unlocking means unlocking the second member from being prohibited in movement by the prohibiting means.

3. The endoscope washer-disinfector according to claim 2, wherein the first member is a driving roller having at least a surface made of rubber and being rotatable, the surface contacting the shaft, and
the second member is a guide member tightly holding the shaft with the driving roller, making the shaft advance and retreat inside along the duct in association with rotation of the driving roller, and having a rubber-made surface to contact the shaft for guiding advancing and retreating actions of the shaft.

4. The endoscope washer-disinfector according to claim 3, wherein
the forcing means is a spring having both ends, one end being fixed and the other end contacting the guide member to push the guide member in the predetermined direction,
the prohibiting member is a plate member having both ends, of which one end is fixed and the other end is detachably latching the guide member, and being formed to prohibit the guide member pushed by the spring from moving in the predetermined direction, and
the unlocking means is a pin moving the plate so that the other end of the plate is removed from the guide member.

5. The endoscope washer-disinfector according to claim 4, wherein the brush cassette has a base portion through which a through-hole is formed to reach an inner chamber accommodating the plate and the driving roller therein, the through-hole being positionally corresponding to the plate, and
the pin is build at the loading part so that the pin touches the plate via the through-hole when the brush cassette is loaded.

6. The endoscope washer-disinfector according to claim 5, wherein the base portion of the brush cassette has a second through-hole formed therethrough so as to positionally correspond to a central position of the driving roller and reach the inner chamber, and
the loading part has a driving shaft built therein, the driving shaft being rotatable by a driving source and being fit with the driving roller via the second through-hole when the brush cassette is loaded.

7. The endoscope washer-disinfector according to claim 2, wherein the prohibiting means is configured to additionally behave as means for prohibiting the second member from returning to the first position, after the second member has moved to the second position in response to an unlocking action of the unlocking means.

8. The endoscope washer-disinfector according to claim 1, wherein the lock device comprises prohibiting means prohibiting the second member from being moved, and
the unlocking commander comprises unlocking means unlocking the second member from being prohibited in movement by the prohibiting means.

9. The endoscope washer-disinfector according to claim 8, wherein the first member is a driving roller having at least a surface made of rubber and being rotatable, the surface contacting the shaft, and
the second member is a guide roller tightly holding the shaft with the driving roller, making the shaft advance and retreat inside along the duct in association with rotation of the driving roller, and having a rubber-made surface to contact the shaft for guiding advancing and retreating actions of the shaft.

10. The endoscope washer-disinfector according to claim 9, wherein the prohibiting means is a spring having both ends and being formed to prohibit the guide roller from moving in the predetermined direction, wherein one of both ends is fixed and the other end thereof is detachably latching the guide member, and
the unlocking means is a shaft member configured to allow the guide roller to escape from a force of the spring by forcing the guide roller in the predetermined direction.

11. The endoscope washer-disinfector according to claim 10, wherein the brush cassette has a base portion through which a through-hole is formed to reach an inner chamber accommodating the plate and the driving roller therein, the through-hole being positionally corresponding to a central position of the guide roller, and
the shaft member is build in the loading part and is formed to allow the shaft to fit with the guide roller via the through-hole when t he brush cassette is loaded and to have a tapered surface giving the operating force to the guide roller in the predetermined direction as t he shaft member passes through the through-hole.

12. The endoscope washer-disinfector according to claim 11, wherein the base portion of the brush cassette has a second through-hole formed therethrough so as to positionally correspond to a central position of the driving roller and reach the inner chamber, and
the loading part has a driving shaft built therein, the driving shaft being rotatable by a driving source and being fit with the driving roller via the second through-hole when the brush cassette is loaded.

13. The endoscope washer-disinfector according to claim 8, wherein the prohibiting means is configured to additionally behave as means for prohibiting the second member from returning to the first position, after the second member has moved to the second position in response to an unlocking action of the unlocking means.

14. The endoscope washer-disinfector according to claim 1, wherein the lock device comprises prohibiting means prohibiting the second member from being moved, and
the unlocking commander comprises an electric mechanism unlocking the second member from being prohibited in movement by the prohibiting means and moving the unlocked second member from the first position to the second position.

15. The endoscope washer-disinfector according to claim 14, wherein the first member is a driving roller having at least a surface made of rubber and being rotatable, the surface contacting the shaft, and
the second member is a guide roller tightly holding the shaft with the driving roller, making the shaft advance and retreat inside along the duct in association with rotation of the driving roller, and having a rubber-made surface to contact the shaft for guiding advancing and retreating actions of the shaft.

16. The endoscope washer-disinfector according to claim 15, wherein the prohibiting means is a spring having both ends and being formed to prohibit the guide roller from moving in the predetermined direction, wherein one of both ends is fixed and the other end thereof is detachably latching the guide member.

17. The endoscope washer-disinfector according to claim 16, wherein the brush cassette has a base portion through which a through-hole is formed to reach an inner chamber accommodating the plate and the driving roller therein, the through-hole being positionally corresponding to a central position of the guide roller, and
the electric mechanism comprises a shaft build in the loading part and formed to pass through the through-hole to fit with the guide roller so that the operating force electrically generated by the electric mechanism is transmitted to the guide roller, when the brush cassette is loaded.

18. The endoscope washer-disinfector according to claim 17, wherein the base portion of the brush cassette has a second through-hole formed therethrough so as to positionally correspond to a central position of the driving roller and reach the inner chamber, and
the loading part has a driving shaft built therein, the driving shaft being rotatable by a driving source and being fit with the driving roller via the second through-hole when the brush cassette is loaded.

19. The endoscope washer-disinfector according to claim 14, wherein the prohibiting means is configured to additionally behave as means for prohibiting the second member from returning to the first position, after the second member has moved to the second position in response to an unlocking action of the unlocking means.

20. The endoscope washer-disinfector according to claim 1, wherein the brush cassette comprises a casing covering at least the first and second members and the lock device and at least part of the casing has a transparent or semitransparent portion which enables a separated condition between the first and second members to be visually observed from outside the casing.

21. A brush cassette detachably loaded to a loading part of an endoscope washer-disinfector for, at least, cleaning and disinfecting an endoscope with a duct, comprising:
a brush element cleaning the duct of the endoscope,
a shaft connected with the brush element;
a first member and a second member formed to hold the shaft tight therebetween and to make the tight held shaft advance and retreat to and from the duct, and
a lock device i) separating the second member from the first member so that the second member is located at a first position and ii) locking the separated second member, when the brush cassette is not loaded on the loading part, while iii) unlocking the locked second member in response to an operating force given responsively to a loading action given to the brush cassette and iv) allowing the second member to be moved to a second position where the first and second members hold tight the shaft therebetween, when the brush cassette is loaded to the loading part.

22. The brush cassette according to claim 21, wherein
the first member is a driving roller having at least a surface made of rubber and being rotatable, the surface contacting the shaft, and
the second member is a guide member tightly holding the shaft with the driving roller, making the shaft advance and retreat inside along the duct in association with rotation of the driving roller, and having a rubber-made surface to contact the shaft for guiding advancing and retreating actions of the shaft.

23. The brush cassette according to claim 1, wherein the lock device comprises
forcing means forcing the second member so as to cause the second member to move from the first position to the second position in a predetermined direction intersecting the shaft, and
prohibiting means prohibiting the second member from being forcibly moved by the forcing means.

24. The brush cassette according to claim 23, wherein the lock device comprises prohibiting means for prohibiting the second member from moving.

25. The brush cassette according to claim 23, wherein the brush cassette comprises a casing covering at least the first and second members and the lock device and at least part of the casing has a transparent or semitransparent portion which enables a separated condition between the first and second members to be visually observed from outside the casing.
